# EUROPEAN PATENT SPECIFICATION

(11) **EP 2 016 053 B1**
(45) Date of publication and mention of the grant of the patent: **10.11.2010**
(21) Application number: 07794404.9
(22) Date of filing: 27.04.2007
(51) Int. Cl.: C07D 209/54, C07C 211/17, C07C 215/20, C07C 255/46

(54) **PROCESS FOR THE SYNTHESIS OF (+) AND (-)-1-ARYL-3-AZABICYCLO[3.1.0]HEXANES**
VERFAHREN ZUR SYNTHESE VON (+) UND (-)-1-ARYL-3-AZABICYCLO[3.1.0]HEXANEN
SYNTHÈSE DE (+) ET (-)-1-ARYL-3-AZABICYCLO[3.1.0]HEXANES

(30) Priority: 28.04.2006 US 796086 P
(43) Date of publication of application: 21.01.2009
(73) Proprietor: Merck Sharp & Dohme Corp., Rahway, NJ 07065 (US)
(72) Inventor: CORLEY, Edward, G., Rahway, New Jersey 07065-0907 (US); MURRY, Jerry, A., Rahway New Jersey, 07065-0900 (US); SIMMONS, Bryon, Rahway, New Jersey 07065-0907 (US); XU, Feng, Rahway, New Jersey 07065-0907 (US)
(74) Representative: Buchan, Gavin MacNicol
(86) International application number: PCT/US2007/010324
(87) International publication number: WO 2007/127421

(56) References cited:
- WO-A-2006/096810
- WO-A-2007/016155
- US-A- 4 196 120
- US-A1- 2004 142 904
- XU FENG ET AL: "Stereocontrolled synthesis of trisubstituted cyclopropanes: expedient, atom-economical, asymmetric syntheses of (+)-Bicifadine and DOV21947." ORGANIC LETTERS 17 AUG 2006, vol. 8, no. 17, 17 August 2006 (2006-08-17), pages 3885-3888, XP002457492 ISSN: 1523-7060
- LANGER P ET AL: "Chemo-, regio-, and diastereoselective synthesis of functionalized cyclopropanes by cyclization of dilithiated nitriles with epibromohydrin." ORGANIC LETTERS 29 NOV 2001, vol. 3, no. 24, 29 November 2001 (2001-11-29), pages 3903-3905, XP002457493 ISSN: 1523-7060
- BONNAUD ET AL: "Stereoselective synthesis of cis and trans 2-substituted 1-phenyl-3-azabicyclo[3.10]hexanes" JOURNAL OF HETEROCYCLIC CHEMISTRY, HETEROCORPORATION. PROVO, US, vol. 30, no. 2, March 1993 (1993-03), pages 505-508, XP002340118 ISSN: 0022-152X
- CASADIO S ET AL: "1 Phenylhydroxymethyl 2 cyclopropane carboxylic acid and derivatives" BOLLETTINO CHIMICO FARMACEUTICO 1978 ITALY, vol. 117, no. 6, 1978, pages 331-342, XP008085513 ISSN: 0006-6648

## Description

### BACKGROUND OF THE INVENTION

The present invention relates to processes for the preparation of (+)-1-aryl-3-azabicyclo[3.1.0]hexanes or a pharmaceutically acceptable salt thereof, and (-)-1-aryl-3-azabicyclo[3.1.0]hexanes or a pharmaceutically acceptable salt thereof. In one embodiment, the present invention relates to processes for the preparation of (+)-1-(4-methylphenyl)-3-azabicyclo[3.1.0]hexane, or a pharmaceutically acceptable salt thereof, and (-)-1-(4-methylphenyl)-3-azabicyclo[3.1.0]hexane or a pharmaceutically acceptable salt thereof. These compounds are known to be useful for treating e.g., pain, depression, anxiety disorders, eating disorders and urinary incontinence (see US Patent Nos. 4,231,935 and 4,196,120 and J. Med. Chem., 1981, 24, 481).

The general processes disclosed in the art for the preparation of racemic, (+) and (-)-1-aryl-3-azabicyclo[3.1.0]hexane result in relatively low and inconsistent yields of the desired product (see e.g., US Patent Nos. 4,118,417, 4,131,611, 4,196,120, 4,231,935, and 4,435,419, PCT Publications WO 2004/012722 and WO 2004/043920, and Epstein, et al., J. Med. Chem., 1981, 24, 481). Some of such processes rely on the use of expensive reagents. In contrast to the previously known processes, the present invention provides effective methodology for the preparation of (+) or (-)-1-aryl-3-azabicyclo[3.1.0]hexanes in relatively high yield and enantiomeric purity. It will be appreciated that (+) or (-)-1-aryl-3-azabicyclo[3.1.0]hexanes, including (+) and (-)-1-(4-methylphenyl)-3-azabicyclo[3.1.0]-hexane are useful therapeutic agents. As such, there is a need for the development of processes for the preparation of (+) and (-)-1-aryl-3-azabicyclo[3.1.0]hexanes which are readily amenable to scale-up, use cost-effective and readily available reagents, and which are therefore capable of practical application to large scale manufacture. Accordingly, the subject invention provides a process for the preparation of (+)-1-aryl-3-azabicyclo[3.1.0]hexanes, (-)-1-aryl-3-azabicyclo[3.1.0]hexanes, including (+)-1-(4-methylphenyl)-3-azabicyclo[3.1.0]hexane and (-)-1-(4-methylphenyl)-3-azabicyclo[3.1.0]hexane via a very simple, short and highly efficient synthesis.

### DETAILED DESCRIPTION OF THE INVENTION

The present invention provides novel processes for preparing a compound of the formula I: 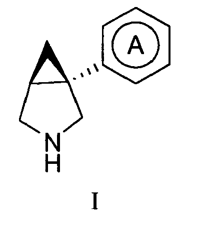 or a pharmaceutically acceptable salt thereof,
wherein the ring A is phenyl, which is unsubstituted or substituted with one or more of a group which is selected from the group consisting of: bromo, chloro, fluoro, iodo, -NO₂, -CN, -NH₂, -NH(C₁₋₈alkyl), -N(C₁₋₈alkyl)₂, -O-C₁₋₈alkyl, C₁₋₈alkyl, C₂₋₈alkenyl, C₂₋₈alkynyl, C₃₋₈ cycloalkyl, and trifluoromethyl, with the proviso that A is not 3,4-dichlorophenyl, comprising:
contacting a phenyl-acetonitrile and (S)-epichlorohydrin of the formula: 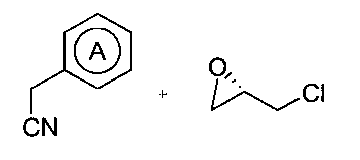 wherein the ring A is phenyl, which is unsubstituted or substituted with one or more of a group which is selected from the group consisting of: bromo, chloro, fluoro, iodo, -NO₂, -CN, -NH₂, -NH(C₁₋₈alkyl), -N(C₁₋₈alkyl)₂, -O-C₁₋₈alkyl, C₁₋₈alkyl, C₂₋₈alkenyl, C₂₋₈alkynyl, C₃₋₈cycloalkyl, and trifluoromethyl, with the proviso that A is not 3,4-dichlorophenyl,
   in the presence of a base,
   to give cyclopropyl compounds of the formula II: 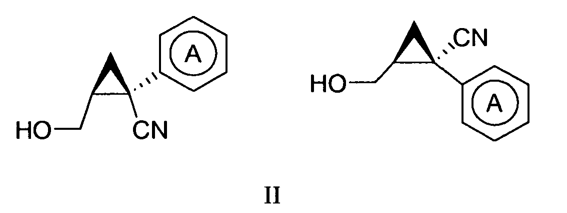 followed by reducing the compounds of formula II with a reducing agent to give amino alcohol compounds of the formula III: 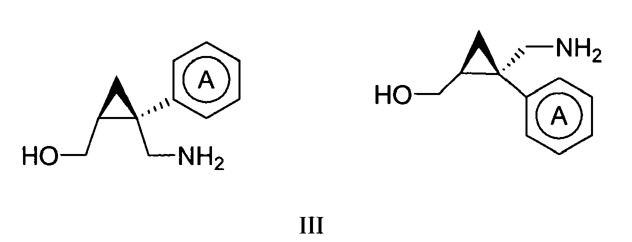 followed by chlorinating the compounds of formula III with a chlorinating agent to give chloro compounds of the formula IV: 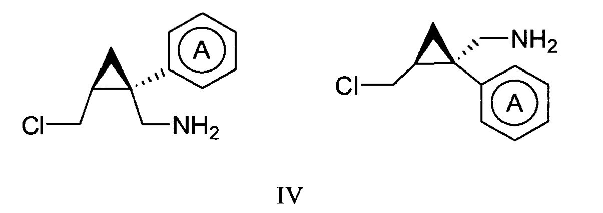 followed by cyclodehydration of the compounds of the formula IV with a base to give the compound of the formula I, or a pharmaceutically acceptable salt thereof;
   wherein the steps are conducted sequentially without isolation of the intermediates.

In an embodiment of the present invention, the ring A is phenyl, which is unsubstituted or substituted with one or more of a group which is selected from the group consisting of: bromo, chloro, dimethylamino, ethyl, methoxy, methyl, and trifluoromethyl, with the proviso that A is not 3,4-dichlorophenyl.

In an embodiment of the present invention, the ring A is selected from the group consisting of:
(1) phenyl,
(2) 4-bromophenyl,
(3) 2-chlorophenyl,
(4) 3-chlorophenyl,
(5) 4-chlorophenyl,
(6) 2,6-dichlorophenyl,
(7) 4-(dimethylamino)phenyl,
(8) 2-methoxyphenyl,
(9) 4-methoxyphenyl,
(10) 4-methylphenyl,
(11) 3-trifluoromethylphenyl, and
(12) 4-trifluoromethylphenyl.

In an embodiment of the present invention, the ring A is 4-methylphenyl.

In one embodiment, the present invention is directed to a process for preparing a compound of the formula I-2: 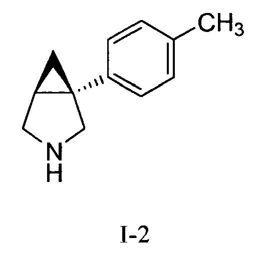 or a pharmaceutically acceptable salt thereof, comprising:
contacting 4-methylphenylacetonitrile and (S)-epichlorohydrin of the formula: 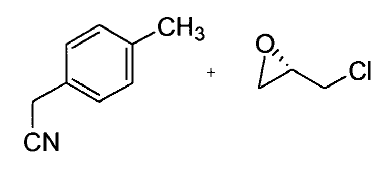 in the presence of a base,
   to give cyclopropyl compounds of the formula II-2: 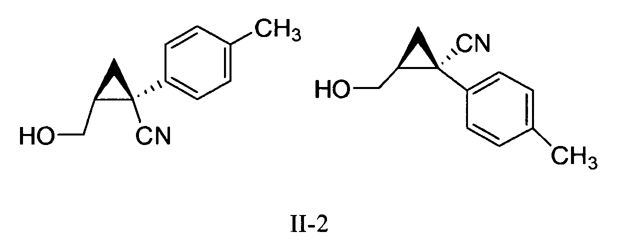
followed by reducing the compounds of formula II with a reducing agent to give amino alcohol compounds of the formula III: 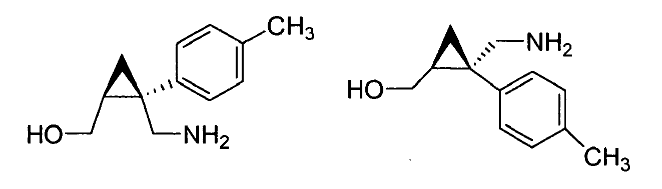 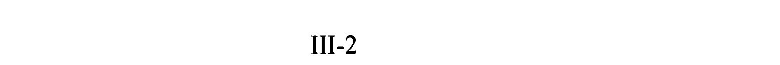 followed by chlorinating the compounds of formula III with a chlorinating agent to give chloro compounds of the formula IV: 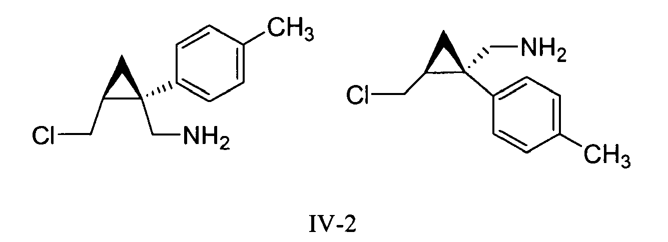 followed by cyclodehydration of the compounds of the formula IV with a base to give the compound of the formula I-2, or a pharmaceutically acceptable salt thereof.

In an embodiment of the present invention the step of contacting the aryl-acetonitrile and (S)-epichlorohydrin [or (R)-epichlorohydrin] in the presence of a base to give cyclopropyl compounds of the formula II [or IIb], the base may be selected from sodium hexamethyldisilazide (NaHMDS), potassium hexamethyldisilazide (KHMDS), lithium hexamethyldisilazide (LiHMDS), potassium t-butoxide, potassium t-pentoxide, potassium amylate, lithium diisopropylamide (LDA), lithium tetramethylpiperidide (LiTMP), sec-butyllithium, or tert-butyllithium, Within this embodiment, the base is selected from sodium hexamethyldisilazide (NaHMDS), potassium hexamethyldisilazide (KHMDS) and lithium hexamethyldisilazide (LiHMDS). Further within this embodiment, the base is sodium hexamethyldisilazide (NaHMDS). Solvents for conducting the step of contacting the aryl-acetonitrile and (S)-epichlorohydrin [or (S)-epichlorohydrin] in the presence of a base to give cyclopropyl compounds of the formula II [or IIb] comprise an organic solvent. Within this embodiment, the organic solvent comprises toluene, tetrahydrofuran (THF), diethyl ether, diglyme, dimethoxyethane (DME), or methyl t-butyl ether. Further within this embodiment, the organic solvent is tetrahydrofuran. The step of contacting the aryl-acetonitrile and (S)-epichlorohydrin [or (S)-epichlorohydrin] in the presence of a base to give cyclopropyl compounds of the formula II [or IIb] is typically carried out at a temperature range of between about -30 and about 25°C. Within this embodiment, the temperature range is less than about 0 °C. Further within this embodiment, the temperature range is between about -20 and about -5 °C.

In an embodiment, of the present invention the step of reducing of the compounds of formula II [or IIb] with a reducing agent to give amino alcohol compounds of the formula III [or IIIb], the reducing agent may be selected from borane dimethyl sulfide complex, borane tetrahydrofuran complex, sodium borohydride-borontrifluoride etherate, a dialkylborane, 9-borabicyclo[3.3.1]nonane (9-BBN), and lithium alumium hydride (LAH). Further within this embodiment, the reducing agent is borane dimethyl sulfide complex. Solvents for conducting the step of reducing of the compounds of formula II with a reducing agent to give amino alcohol compounds of the formula III [or IIIb] comprise an organic solvent. Within this embodiment, the organic solvent comprises toluene, tetrahydrofuran (THF), dimethyl ether, diglyme, dimethoxyethane (DME), or methyl t-butyl ether. Further within this embodiment, the organic solvent is tetrahydrofuran. The step of reducing of the compounds of formula II [or IIb] with a reducing agent to give amino alcohol compounds of the formula III [or IIIb] is typically carried out at a temperature range of between about -30 and about 25 °C. Within this embodiment, the temperature range is less than about 0 °C. Further within this embodiment, the temperature range is between about -20 and about -5 °C.

In an embodiment of the present invention the step of chlorinating the compounds of formula III [or IIIb] with a chlorinating agent to give chloro compounds of the formula IV[or IVb], the chlorinating agent may be selected from thionyl chloride, SO₂Cl₂, and Ph₃P/CCl₄. Further within this embodiment, the chlorinating agent is thionyl chloride. Solvents for conducting the step of chlorinating the compounds of formula III [or IIIb] with a chlorinating agent to give chloro compounds of the formula IV [or IVb] comprise an organic solvent. Within this embodiment, the organic solvent comprises toluene, tetrahydrofuran (THF), diethyl ether, diglyme, dimethoxyethane (DME), methyl t-butyl ether, ethyl acetate, isopropyl acetate or N-methyl pyrrolidinone. Further within this embodiment, the organic solvent comprises tetrahydrofuran, dimethoxyethane and isopropyl acetate. The step of chlorinating the compounds of formula III [or IIIb] with a chlorinating agent to give chloro compounds of the formula IV [or IVb]is typically carried out at a temperature range of between about 0 and about 40 °C. Within this embodiment, the temperature range is less than about 0 °C. Further within this embodiment, the temperature is about 25 °C.

In an embodiment of the present invention the step of cyclodehydration of the compounds of the formula IV [or IVb] with a base to give the compound of formula [or Ib], the base may be selected from sodium hydroxide, potassium hydroxide, potassium bicarbonate, sodium bicarbonate, potassium carbonate, sodium carbonate, Et₃N, i-Pr₂NEt, DABCO, DBU, or other amine bases. Further within this embodiment, the base is sodium hydroxide. Solvents for conducting the step of cyclodehydration of the compounds of the formula IV [or IVb] with a base to give the compound of formula I [or Ib] comprise an aqueous solvent. In the step of cyclodehydration of the compounds of the formula IV [or IVb] with a base to give the compound of formula I [or Ib], the pH is typically at a range of between about 7-10. Within this embodiment, the pH is about 8-10. Further within this embodiment, the the pH is about 8.5-9.5.

In a further embodiment, the present invention is directed to a process for the preparation of (+)-1-(4-methylphenyl)-3-azabicyclo[3.1.0]hexane as depicted below:

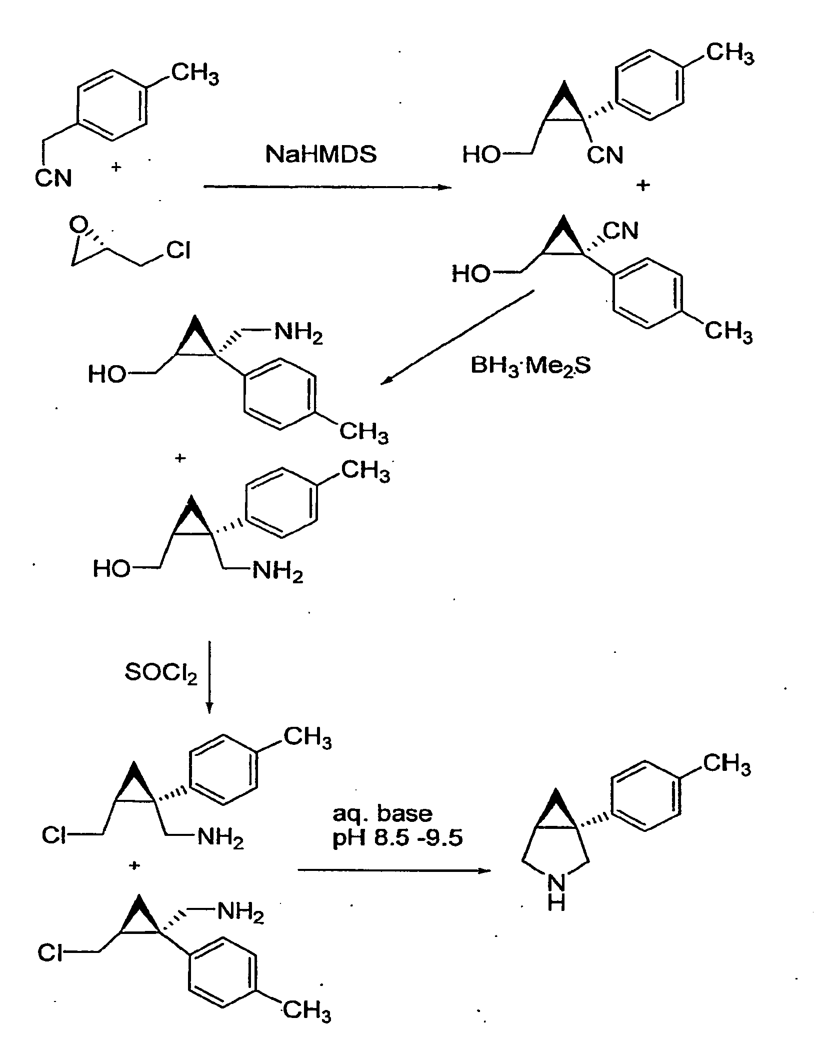

In a further embodiment, the present invention is directed to a process for the preparation of (+)-1-(4-methylphenyl)-3-azabicyclo[3.1.0]hexane as depicted below:

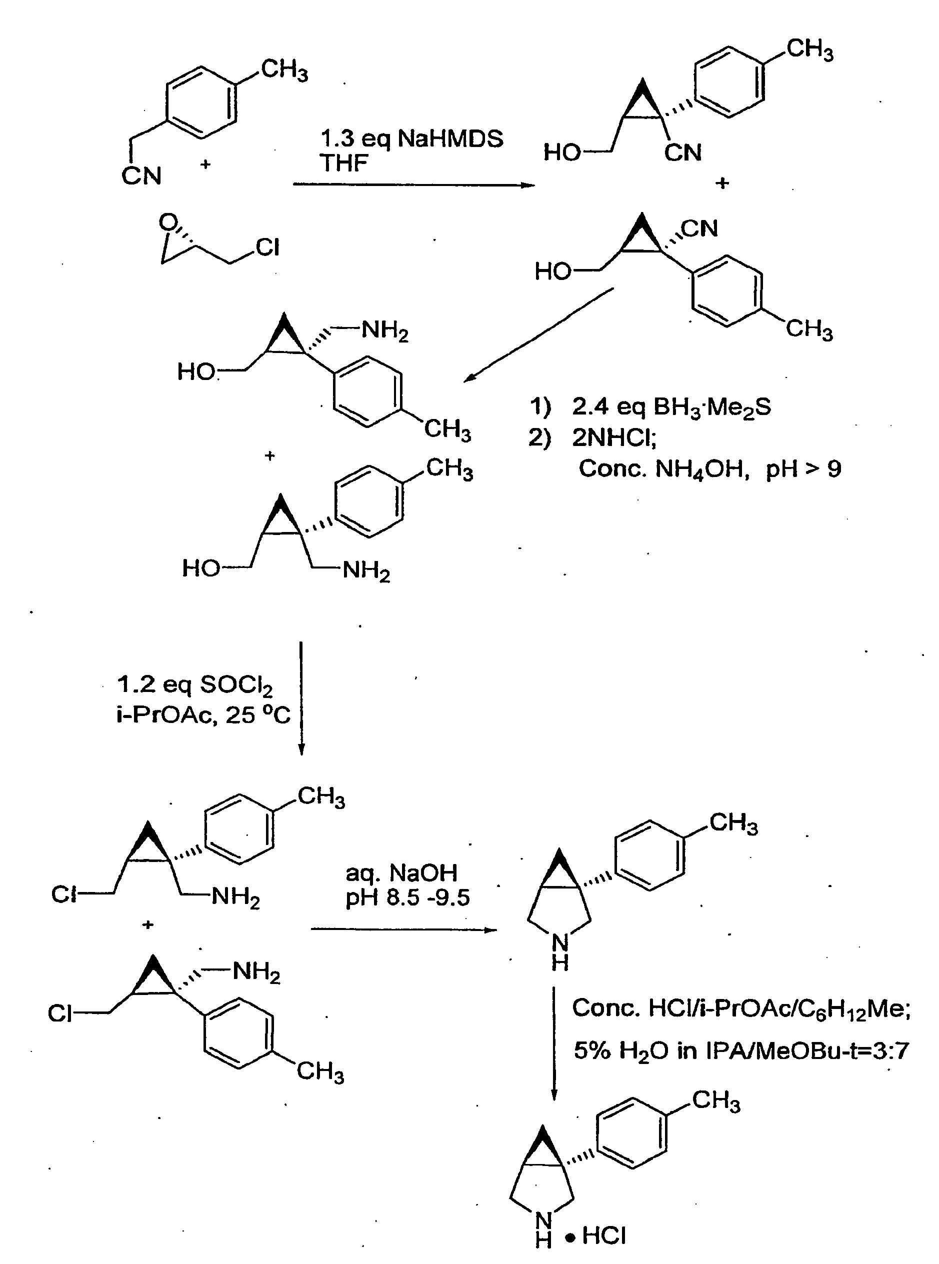

The present invention provides a heavy metal-free synthesis that is efficient and atom economic so that (+)-1-(4-methylphenyl)-3-azabicyclo[3.1.0]hexane or (-)-1-(4-methylphenyl)-3-azabicyclo[3.1.0]hexane may be prepared via a single through process without requiring isolation of any intermediates. Starting from inexpensive, commercially available 4-methylphenylacetonitrile and (S)-epichlorohydrin (or (R)-epichlorohydrin), the key cyclopropane intermediate is constructed. Without further workup, the crude reaction mixture is reduced with borane dimethyl sulfide complex in one pot to afford the amino alcohol intermediates. The desired cis amino alcohol is directly cylodehydrated to give (+)-1-(4-methylphenyl)-3-azabicyclo[3.1.0]hexane or (-)-1-(4-methylphenyl)-3-azabicyclo[3.1.0]hexane. The whole synthesis may be conducted as a single stage through process to allow direct isolation of (+)-1-(4-methylphenyl)-3-azabicyclo[3.1.0]hexane HCl salt or (-)-1-(4-methylphenyl)-3-azabicyclo[3.1.0]-hexane HCl salt.

Another aspect of this invention is directed to the foregoing precesses wherein the (+)-1-(4-methylphenyl)-3-azabicyclo[3.1.0]hexane, or a pharmaceutically acceptable salt thereof, is present in an enantiomeric purity (enantiomeric excess) of greater than 90%, greater than 95%, greater than 98%, greater than 99%, greater than 99.5% (enantiomeric excess) or greater than 99.9% (enantiomeric excess).

Another aspect of this invention is directed to the foregoing precesses wherein the (-)-1-(4-methylphenyl)-3-azabicyclo[3.1.0]hexane, or a pharmaceutically acceptable salt thereof, is present in an enantiomeric purity (enantiomeric excess) of greater than 90%, greater than 95%, greater than 98%, greater than 99%, greater than 99.5% (enantiomeric excess) or greater than 99.9% (enantiomeric excess).

The term "pharmaceutically acceptable salts" refers to salts prepared from pharmaceutically acceptable non-toxic acids including inorganic or organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like. Specific acids include citric, hydrobromic, hydrochloric, maleic, phosphoric, sulfuric, fumaric, and tartaric acids. A specific acid is hydrochloric acid.

The present process is surprisingly efficient, minimizing the production of side products, and increasing productivity and purity. The starting materials and reagents for the subject processes are either commercially available or are known in the literature or may be prepared following literature methods described for analogous compounds. The skills required in carrying out the reaction and purification of the resulting reaction products are known to these in the art. Purification procedures include crystallization, distillation, normal phase or reverse phase chromatography.

The following examples are provided for the purpose of further illustration only and are not intended to be limitations on the disclosed invention. Unless otherwise noted, all reactions were conducted under N₂ atmosphere using standard air-free manipulation techniques. Solvents were purchased from Fisher Scientific Company and used without further purification. Commercial reagents were purchased either from Aldrich or Bayer and used without further purification. High performance liquid chromatography (HPLC) analysis was performed using Agilent Technology 1100 series instrument with ACE 5 C18 (240 x 4.6 mm I.D., 5 µm particle size) column. Proton nuclear magnetic resonance (¹H NMR) spectra were measured on Bruker Avance-400 instrument (400 MHz). Carbon nuclear magnetic resonance (¹³C NMR) spectra were measured on Bruker Avance-400 instrument (100 MHz) with complete proton decoupling. Chemical shifts are reported in ppm downfield from tetramethylsilane (TMS).

### EXAMPLE 1

### (1R,5S)-(+)-1-(4-Methylphenyl)-3-azabicyclo[3,10]hexane [(+)-Bicifadine]

To a solution of 4-methylphenylacetonitrile (100 g) and S-(+)-epichlorohydrin (86.4 g) in MeOBu-t (800 mL) between -25 to -30 °C under atmosphere of N₂ was added LiHMDS (971 mL, 1M in hexane) dropwise over 2 h. After aging additional 2 h at -30 °C, the reaction mixture was quenched into 5N HCl (448 mL) while the temperature was maintained below 20 °C. After phase separation, the organic phase was washed with water (200 mL), azetropically dried and solvent-switched to DME (ca. 1 L) in vacuum. The above crude chlorohydrin in DME cooled to -15 °C. NaHMDS solution (747 mL, 2M in THF) was added dropwise over 2 h. The reaction mixture was aged for additional 1 h between -15 to -20 °C. BH₃-Me₂S (neat, 10M, 224 mL) was added dropwise over 1-2 h while the internal temperature was kept below 10 °C. The reaction mixture was then gradually warmed to 40 °C over 2 h and aged for additional 3 h at 40 °C. The reaction mixture was cooled to 20-25 °C and slowly quenched into a 4N HCl (934 mL) solution. The mixture was then aged for 1 h at 40 °C. Ammonium hydroxide (401 mL) and i-PrOAc (500mL) was added. The aqueous phase was back extracted with i-PrOAc (400 mL). The combined organic phase was washed with water (100 ml x 2), azetropically dried and solvent-switched to i-PrOAc in vacuum (ca. 800 mL).

The above crude amino alcohol solution in i-PrOAc was slowly subsurface-added to a solution of SOCl₂ (0.911 mol, 66.5 mL) in i-PrOAc (450 mL) at ambient temperature over 2 h. After aging additional 1-3 h, 5.0 N NaOH (674 mL) was added over 1 h while the batch temperature was maintained at <30 °C with external cooling. The two-phase reaction mixture was stirred for 1 h at ambient temperature to allow pH to stabilize (usually to 8.5-9.0) with NaOH pH titration. The organic phase was washed with water (2 x 100 mL). Conc. HCl (68.5 mL) was added to the organic phases while the internal temperature was kept <30 °C. The aqueous i-PrOAc was azeotropically concentrated in vacuum to a final volume of ca. 500 mL. Methylcyclohexane (350 mL) was added slowly over 2 h. The wet cake was displacement washed with 150 mL of 45% methylcyclohexan/i-PrOAc followed by a slurry wash (150 mL, i-PrOAc) and a displacement wash (150 mL, i-PrOAc). Typically isolated in yield: 65% over 4 steps, in >99 ee%.

(1R,5S)-(+)-1-(4-Methylphenyl)-3-azabicyclo[3,10]hexane HCl salt (100 g) was dissolved in a solution of i-PrOH (285 mL) and water (15 mL) at 75 °C. Seeds (1 g) was added and the batch was allowed to cool to ambient temperature over 2-4 h. MeOBu-t (700 mL) was then added dropwise over 2 h. After aging 1 h at 20 °C, the slurry was filtered. The wet cake was displacement washed with 150 mL of 30% i-PrOH in MeOBu-t followed by 2x150 mL 10% i-PrOH in MeOBu-t (slurry wash, then displacement wash). The (1R,3S)-(+)-1-(4-methylphenyl)-3-azabicyclo[3,10]hexane HCl salt was suction dried under N₂ at ambient temperature. Typical yield: 95%. ¹H-NMR (400 MHz, d₄-MeOH): δ 7.17 (m, 4H), 3.73 (d, J = 11.4 Hz, 1 H), 3.66 (dd, J = 3.8, 11.4 Hz, 1 H), 3.58 (d, J = 11.4 Hz, 1 H), 3.51 (d, J = 11.4 Hz, 1 H), 2.31 (s, 3 H), 2.10 (m, 1H), 1.22 (t, J = 7.5 Hz, 1 H), 1.12 (t, J = 5.4 Hz, 1 H). ¹³C-NMR (100 MHz, d₄-MeOH): δ 138.3, 136.8, 130.6, 128.3, 52.3, 49.3, 32.3, 24.2, 21.2, 16.0. Anal. Calcd for C-₁₂H₁₆ClN: C, 68.73; H, 7.69; N, 6.68. Found: C, 68.52; H, 7.69; N, 6.64.

## Claims

1. A process for preparing a compound of the formula I: 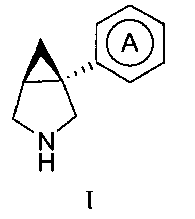 or a pharmaceutically acceptable salt thereof,
wherein the ring A is phenyl, which is unsubstituted or substituted with one or more of a group which is selected from the group consisting of: bromo, chloro, fluoro, iodo, -NO₂, -CN, -NH₂, -NH(C₁₋₈alkyl), -N(C₁₋₈alkyl)₂, -O-C₁₋₈akyl, C₁₋₈alkyl, C₂₋₈alkenyl, C₂₋₈alkynyl, C₃₋₈cycloalkyl, and trifluoromethyl, with the proviso that A is not 3,4-dichlorophenyl,
comprising:
contacting a phenyl-acetonitrile and (S)-epichlorohydrin of the formula: 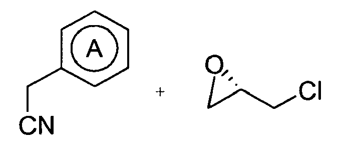 wherein the ring A is phenyl, which is unsubstituted or substituted with one or more of a group which is selected from the group consisting of: bromo, chloro, fluoro, iodo, -NO₂, -CN, -NH₂, -NH(C₁₋₈alkyl), -N(C₁₋₈alkyl)₂, -O-C₁₋₈alkyl, C₁₋₈akyl, C₂₋₈alkenyl, C₂₋₈alkynyl, C₃₋₈cycloalkyl, and trifluoromethyl, with the proviso that A is not 3,4-dichlorophenyl,
in the presence of a base,
to give cyclopropyl compounds of the formula II: 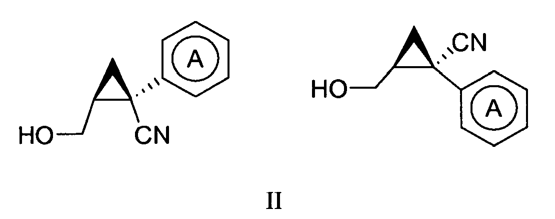 followed by reducing the compounds of formula II with a reducing agent to give amino alcohol compounds of the formula III: 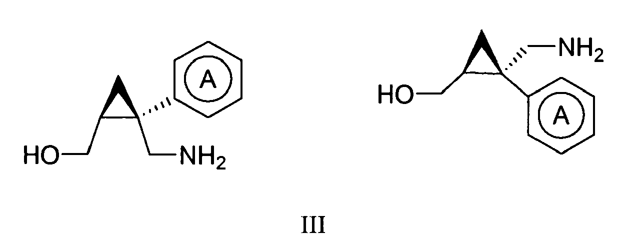 followed by chlorinating the compounds of formula III with a chlorinating agent to give chloro compounds of the formula IV: 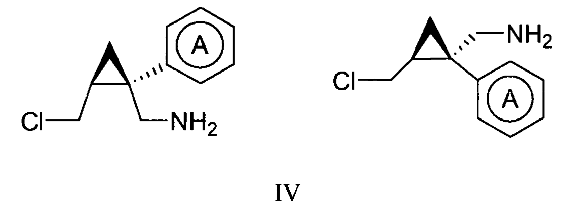 followed by cyclodehydration of the compounds of the formula IV with a base to give the compound of the formula I, or a pharmaceutically acceptable salt thereof;
wherein the steps are conducted sequentially without isolation of the intermediate compounds.

2. A process according to claim 1 for preparing a compound of the formula I-2: 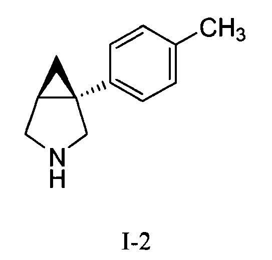 or a pharmaceutically acceptable salt thereof,
comprising:
contacting 4-methylphenylacetonitrile and (S)-epichlorohydrin of the formula: 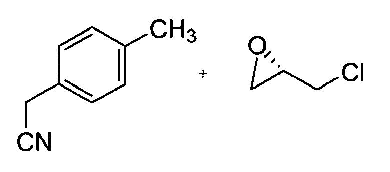 in the presence of a base,
to give cyclopropyl compounds of the formula II-2: 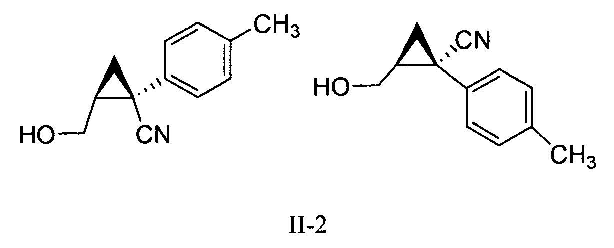 followed by reducing the compounds of formula II with a reducing agent to give amino alcohol compounds of the formula III: 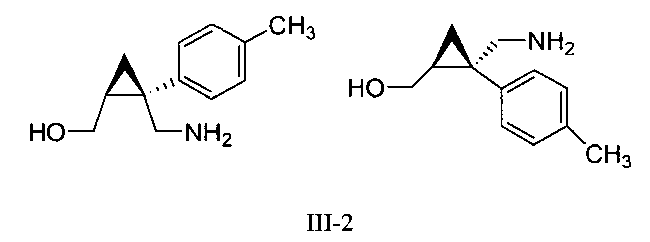 followed by chlorinating the compounds of formula III with a chlorinating agent to give chloro compounds of the formula IV: 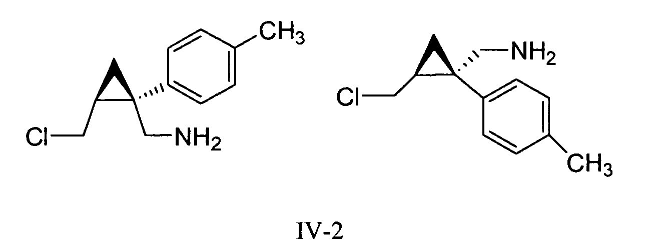 followed by cyclodehydration of the compounds of the formula IV with a base to give the compound of the formula I-2, or a pharmaceutically acceptable salt thereof.

3. The process of Claim 1 or Claim 2 wherein the step of contacting the phenyl-acetonitrile, or the 4-methylphenylacetonitrile, and (S)-epichlorohydrin in the presence of a base to give cyclopropyl compounds of the formula II, the base is selected from sodium hexamethyldisilazide (NaHMDS), potassium hexamethyldisilazide (KHMDS), lithium hexamethyldisilazide (LiHMDS), potassium t-butoxide, potassium t-pentoxide, potassium amylate, lithium diisopropylamide (LDA), lithium tetramethylpiperidide (LiTMP), sec-butyllithium, and tert-butyllithium.

4. The process of Claim 3 wherein the base is selected from sodium hexamethyldisilazide (NaHMDS), potassium hexamethyldisilazide (KHMDS) and lithium hexamethyldisilazide (LiHMDS).

5. The process of Claim 4 wherein the base is sodium hexamethyldisilazide (NaHMDS).

6. The process of Claim 1 or Claim 2 wherein the step of reducing of the compounds of the formula II, or the formula II-2, with a reducing agent to give amino alcohol compounds of the formula III, or the formula III-2, the reducing agent is selected from borane dimethyl sulfide complex, borane tetrahydrofuran complex, sodium borohydride-borontrifluoride etherate, a dialkylborane, 9-borabicyclo[3.3.1]nonane (9-BBN), and lithium alumium hydride (LAH).

7. The process of Claim 6 wherein the reducing agent is borane dimethyl sulfide complex.

8. The process of Claim 1 or Claim 2 wherein the step of chlorinating the compounds of the formula III, or the formula III-2, with a chlorinating agent to give chloro compounds of the formula IV, or the formula IV-2, , the chlorinating agent is selected from thionyl chloride, SO₂Cl₂, and Ph₃P/CCl₄.

9. The process of Claim 8 wherein the chlorinating agent is thionyl chloride.

10. The process of Claim 1 or Claim 2 wherein the step of cyclodehydration of the compounds of the formula IV, or the formula IV-2, with a base to give the compound of formula I, or the formula I-2, the base is selected from sodium hydroxide, potassium hydroxide, potassium bicarbonate, sodium bicarbonate, potassium carbonate, sodium carbonate, Et₃N, i-Pr₂NEt, DABCO and DBU.

11. The process of Claim 1 wherein the ring A is selected from the group consisting of:
(1) phenyl,
(2) 4-bromophenyl,
(3) 2-chlorophenyl,
(4) 3-chlorophenyl,
(5) 4-chlorophenyl,
(6) 2,6-dichlorophenyl,
(7) 4-(dimethylamino)phenyl,
(8) 2-methoxyphenyl,
(9) 4-methoxyphenyl,
(10) 4-methylphenyl,
(11) 3-trifluoromethylphenyl, and
(12) 4-trifluoromethylphenyl.

## Patentansprüche

1. Ein Verfahren zur Herstellung einer Verbindung der Formel I: 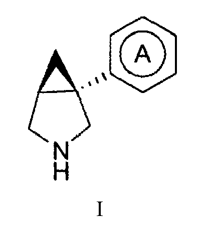 oder eines pharmazeutisch annehmbaren Salzes davon,
wobei der Ring A Phenyl ist, das unsubstituiert oder substituiert ist mit einer oder mehreren Gruppen, ausgewählt aus der Gruppe bestehend aus: Brom, Chlor, Fluor, Iod, -NO₂, -CN, -NH₂, -NH(C₁₋₈-Alkyl), -N(C₁₋₈-Alkyl)₂, -O-C₁₋₈-Alkyl, C₁₋₈-Alkyl, C₂₋₈-Alkenyl, C₂₋₈-Alkinyl, C₃₋₈-Cycloalkyl und Trifluormethyl, mit der Maßgabe, dass A nicht 3,4-Dichlorphenyl ist,
umfassend:
Inkontaktbringen eines Phenylacetonitrils und (S)-Epichlorhydrin der Formel: 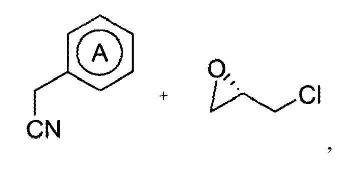 wobei der Ring A Phenyl ist, das unsubstituiert oder substituiert ist mit einer oder mehreren Gruppen, ausgewählt aus der Gruppe bestehend aus: Brom, Chlor, Fluor, Iod, -NO₂, -CN, -NH₂, -NH(C₁₋₈-Alkyl), -N(C₁₋₈-Alkyl)₂, -O-C₁₋₈-Alkyl, C₁₋₈-Alkyl, C₂₋₈-Alkenyl, C₂₋₈-Alkinyl, C₃₋₈-Cycloalkyl und Trifluormethyl, mit der Maßgabe, dass A nicht 3,4-Dichlorphenyl ist,
in Gegenwart einer Base,
um Cyclopropylverbindungen der Formel II zu ergeben: 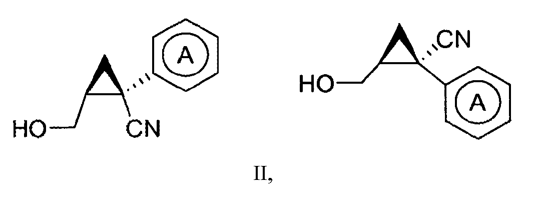 gefolgt von der Reduktion der Verbindungen der Formel II mit einem Reduktionsmittel, um Aminoalkoholverbindungen der Formel III zu ergeben: 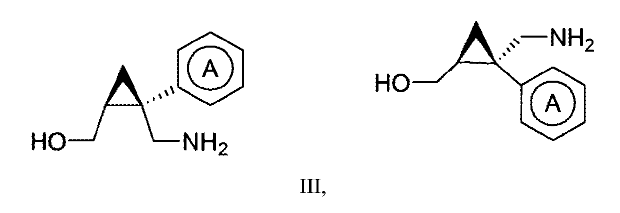 gefolgt von der Chlorierung der Verbindungen der Formel III mit einem Chlorierungsmittel, um Chlorverbindungen der Formel IV zu ergeben: 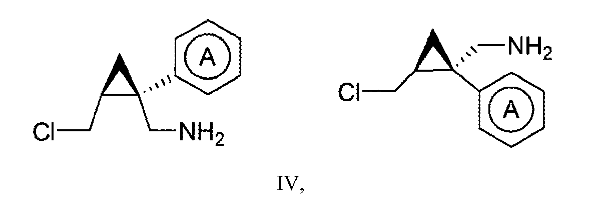 gefolgt von der Cyclodehydratisierung der Verbindungen der Formel IV mit einer Base, um die Verbindung der Formel I oder ein pharmazeutisch annehmbares Salz davon zu ergeben,
wobei die Schritte der Reihe nach ohne Isolierung der Zwischenverbindungen durchgeführt werden.

2. Ein Verfahren gemäß Anspruch 1 zur Herstellung einer Verbindung der Formel I-2: 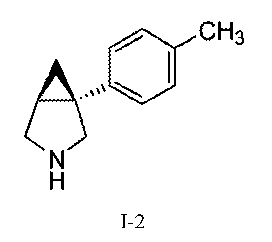 oder eines pharmazeutisch annehmbaren Salzes davon,
umfassend:
Inkontaktbringen von 4-Methylphenylacetonitril und (S)-Epichlorhydrin der Formel: 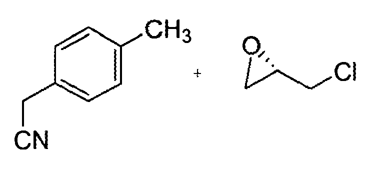 in Gegenwart einer Base,
um Cyclopropylverbindungen der Formel II-2 zu ergeben: 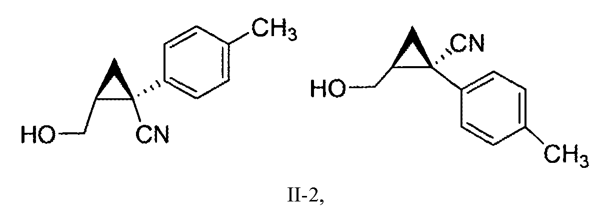 gefolgt von der Reduktion der Verbindungen der Formel II mit einem Reduktionsmittel, um Aminoalkoholverbindungen der Formel III zu ergeben: 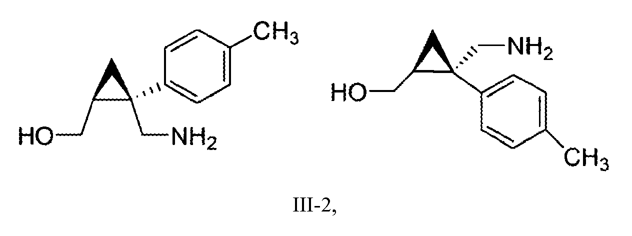 gefolgt von der Chlorierung der Verbindungen der Formel III mit einem Chlorierungsmittel, um Chlorverbindungen der Formel IV zu ergeben: 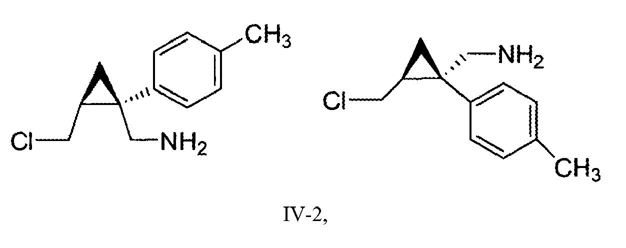 gefolgt von der Cyclodehydratisierung der Verbindungen der Formel IV mit einer Base, um die Verbindung der Formel 1-2 oder ein pharmazeutisch annehmbares Salz davon zu ergeben.

3. Das Verfahren gemäß Anspruch 1 oder Anspruch 2, wobei bei dem Schritt des Inkontaktbringens des Phenylacetonitrils oder des 4-Methylphenylacetonitrils und (S)-Epichlorhydrin in Gegenwart einer Base zur Bildung von Cyclopropylverbindungen der Formel II die Base ausgewählt ist aus Natriumhexamethyldisilazid (NaHMDS), Kaliumhexamethyldisilazid (KHMDS), Lithiumhexamethyldisilazid (LiHMDS), Kalium-t-butoxid, Kalium-t-pentoxid, Kaliumamylat, Lithiumdiisopropylamid (LDA), Lithiumtetramethylpiperidid (LiTMP), sek-Butyllithium und tert-Butyllithium.

4. Das Verfahren gemäß Anspruch 3, wobei die Base ausgewählt ist aus Natriumhexamethyldisilazid (NaHMDS), Kaliumhexamethyldisilazid (KHMDS) und Lithiumhexamethyldisilazid (LiHMDS).

5. Das Verfahren gemäß Anspruch 4, wobei die Base Natriumhexamethyldisilazid (NaHMDS) ist.

6. Das Verfahren gemäß Anspruch 1 oder Anspruch 2, wobei bei dem Schritt der Reduktion der Verbindungen der Formel II oder der Formel II-2 mit einem Reduktionsmittel zur Bildung von Aminoalkoholverbindungen der Formel III oder der Formel III-2 das Reduktionsmittel ausgewählt ist aus Boran-Dimethylsulfid-Komplex, Boran-Tetrahydrofuran-Komplex, Natriumborhydrid-Bortrifluorid-Etherat, einem Dialkylboran, 9-Borabicyclo[3.3.1]nonan (9-BBN) und Lithiumaluminiumhydrid (LAH).

7. Das Verfahren gemäß Anspruch 6, wobei das Reduktionsmittel Boran-Dimethylsulfid-Komplex ist.

8. Das Verfahren gemäß Anspruch 1 oder Anspruch 2, wobei bei dem Schritt der Chlorierung der Verbindungen der Formel III oder der Formel III-2 mit einem Chlorierungsmittel zur Bildung von Chlorverbindungen der Formel IV oder der Formel IV-2 das Chlorierungsmittel ausgewählt ist aus Thionylchlorid, SO₂Cl₂ und Ph₃P/CCl₄.

9. Das Verfahren gemäß Anspruch 8, wobei das Chlorierungsmittel Thionylchlorid ist.

10. Das Verfahren gemäß Anspruch 1 oder Anspruch 2, wobei bei dem Schritt der Cyclodehydratisierung der Verbindungen der Formel IV oder der Formel IV-2 mit einer Base zur Bildung der Verbindung der Formel I oder der Formel 1-2 die Base ausgewählt ist aus Natriumhydroxid, Kaliumhydroxid, Kaliumhydrogencarbonat, Natriumhydrogencarbonat, Kaliumcarbonat, Natriumcarbonat, Et₃N, i-Pr₂NEt, DABCO und DBU.

11. Das Verfahren gemäß Anspruch 1, wobei der Ring A ausgewählt ist aus der Gruppe, bestehend aus:
(1) Phenyl,
(2) 4-Bromphenyl,
(3) 2-Chlorphenyl,
(4) 3-Chlorphenyl,
(5) 4-Chlorphenyl,
(6) 2,6-Dichlorphenyl,
(7) 4-(Dimethylamino)phenyl,
(8) 2-Methoxyphenyl,
(9) 4-Methoxyphenyl,
(10) 4-Methylphenyl,
(11) 3-Trifluormethylphenyl und
(12) 4-Trifluormethylphenyl.

## Revendications

1. Procédé pour la préparation d'un composé de la formule I: 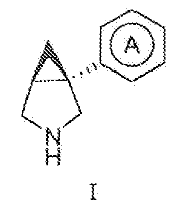 ou d'un sel pharmaceutiquement acceptable de celui-ci,
dans lequel le cycle A est un phényle, qui est non substitué ou substitué avec un ou plusieurs d'un groupe qui est choisi dans le groupe constitué de: un bromo, un chloro, un fluoro, un iodo, -NO₂, -CN, -NH₂, un -NH(alkyleC₁₋₈), un -N(alkyle C₁₋₈)₂, un -O-alkyle C₁₋₈, un alkyle C₁₋₈, un alcényle C₂₋₈, un alcynyle C₂₋₈, un cycloalkyle C₃₋₈ et un trifluorométhyle, sous réserve que A ne soit pas un 3,4-dichlorophényle,
comprenant:
la mise en contact d'un phényl-acétonitrile et d'une (S)-épichlorohydrine de la formule: 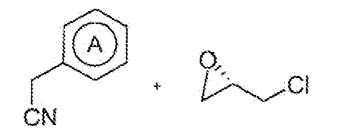 dans lequel le cycle A est un phényle, qui est non substitué ou substitué avec un ou plusieurs d'un groupe qui est choisi dans le groupe constitué de: un bromo, un chloro, un fluoro, un iodo, -NO₂, -CN, -NH₂, un -NH(alkyle C₁₋₈), un -N(alkyle C₁₋₈)₂, un -O-alkyle C₁₋₈, un alkyle C₁₋₈, un alcényle C₂₋₈, un alcynyle C₂₋₈, un cycloalkyle C₃₋₈ et un trifluorométhyle, sous réserve que A ne soit pas un 3,4-dichlorophényle,
en présence d'une base,
pour donner des composés de cyclopropyle de la formule II: 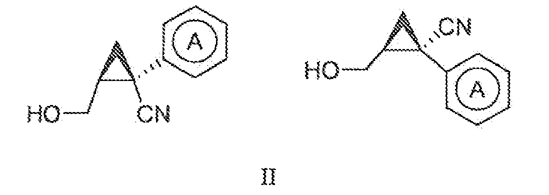 suivie par la réduction des composés de la formule II avec un agent réducteur pour donner des composés d'aminoalcool de la formule III: 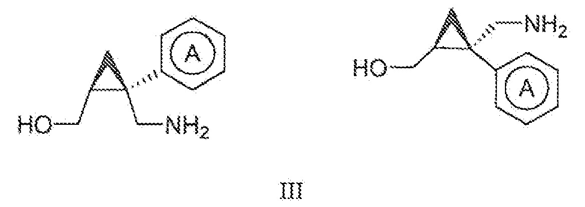 suivie par la chloration des composés de la formule III avec un agent de chlorant pour donner des composés de chloro de la formule IV: 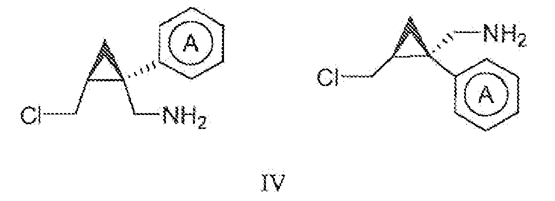 suivie par la cyclodéshydratation des composés de la formule IV avec une base pour donner le composé de la formule I, ou un sel pharmaceutiquement acceptable de celui-ci;
dans lequel les étapes sont conduites séquentiellement sans isolement des composés intermédiaires.

2. Procédé selon la revendication 1 pour la préparation d'un composé de la formule I-2: 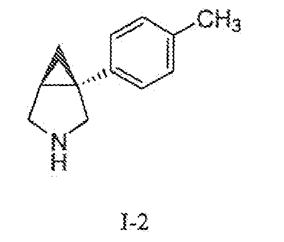 ou d'un sel pharmaceutiquement acceptable de celui-ci,
comprenant:
la mise en contact d'un 4-méthylphénylacétonitrile et d'une (S)-épichlorohydrine de la formule: 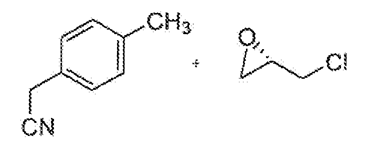 en présence d'une base,
pour donner des composés de cyclopropyle de la formule II-2: 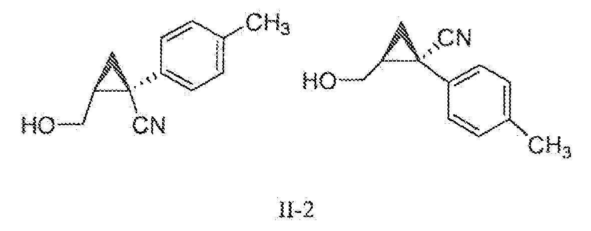 suivie par la réduction des composés de la formule II avec un agent réducteur pour donner des composés d'aminoalcool de la formule III: 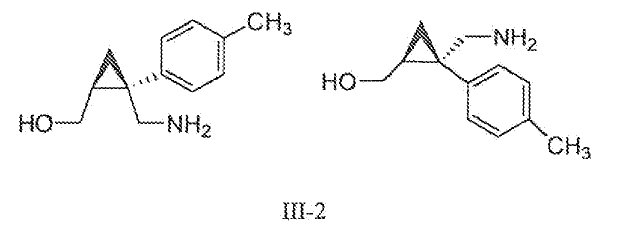 suivie par la chloration des composés de la formule III avec un agent chlorant pour donner des composés de chloro de la formule IV: 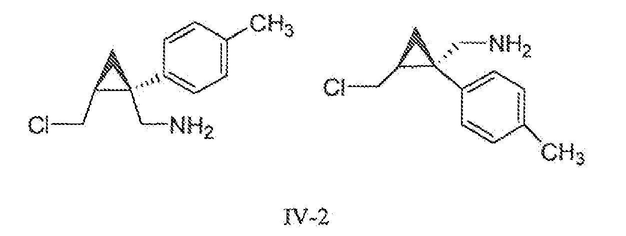 suivie par la cyclodéshydratation des composés de la formule IV avec une base pour donner le composé de la formule 1-2, ou un sel pharmaceutiquement acceptable de celui-ci.

3. Procédé selon la revendication 1 ou la revendication 2, dans lequel, dans l'étape de mise en contact du phényl-acétonitrile, ou du 4-méthylphénylacétonitrile, et de la (S)-épichlorohydrine en présence d'une base pour donner des composés de cyclopropyle de la formule II, la base est choisie parmi l'hexaméthyldisilazide de sodium (NaHMDS), l'hexaméthyldisilazide de potassium (KHMDS), l'hexaméthyldisilazide de lithium (LiHMDS), le t-butoxyde de potassium, le t-pentoxyde de potassium, l'amylate de potassium, le diisopropylamide de lithium (LDA), le tétraméthylpipéridide de lithium (LiTMP), le sec-butyllithium et le tert-butyllithium.

4. Procédé selon la revendication 3, dans lequel la base est choisie parmi l'hexaméthyldisilazide de sodium (NaHMDS), l'hexaméthyldisilazide de potassium (KHMDS) et l'hexaméthyldisilazide de lithium (LiHMDS).

5. Procédé selon la revendication 4, dans lequel la base est l'hexaméthyldisilazide de sodium (NaHMDS).

6. Procédé selon la revendication 1 ou la revendication 2, dans lequel, dans l'étape de réduction des composés de la formule II, ou de la formule II-2, avec un agent réducteur pour donner des composés d'aminoalcool de la formule III, ou de la formule III-2, l'agent réducteur est choisi parmi un complexe de diméthylsulfure borane, un complexe de tétrahydrofurane borane, un borohydrure de sodium-éthérate de trifluorure de bore, un dialkylborane, le 9-borabicyclo[3.3.1]nonane (9-BBN) et l'hydrure de lithium et d'aluminium (LAH).

7. Procédé selon la revendication 6, dans lequel l'agent réducteur est un complexe de diméthylsulfure borane.

8. Procédé selon la revendication 1 ou la revendication 2, dans lequel, dans l'étape de chloration des composés de la formule III, ou de la formule III-2, avec un agent chlorant pour donner des composés de chloro de la formule IV, ou de la formule IV-2, l'agent chlorant est choisi parmi le chlorure de thionyle, SO₂Cl₂ et Ph₃P/CCl₄.

9. Procédé selon la revendication 8, dans lequel l'agent chlorant est le chlorure de thionyle.

10. Procédé selon la revendication 1 ou la revendication 2, dans lequel, dans l'étape de cyclodéshydratation des composés de la formule IV, ou de la formule IV-2, avec une base pour donner le composé de la formule I, ou de la formule 1-2, la base est choisie parmi l'hydroxyde de sodium, l'hydroxyde de potassium, le bicarbonate de potassium, le bicarbonate de sodium, le carbonate de potassium, le carbonate de sodium, Et₃N, i-Pr₂NEt, DABCO et DBU.

11. Procédé selon la revendication 1, dans lequel le cycle A est choisi dans le groupe constitué de:
(1) un phényle,
(2) un 4-bromophényle,
(3) un 2-chlorophényle,
(4) un 3-chlorophényle,
(5) un 4-chlorophényle,
(6) un 2,6-dichlorophényle,
(7) un 4-(diméthylamino)phényle,
(8) un 2-méthoxyphényle,
(9) un 4-méthoxyphényle,
(10) un 4-méthylphényle,
(11) un 3-trifluorométhylphényle, et
(12) un 4-trifluorométhylphényle.
